# EUROPEAN PATENT SPECIFICATION

(11) **EP 0 919 246 B1**
(45) Date of publication and mention of the grant of the patent: **24.04.2002**
(21) Application number: 97830555.5
(22) Date of filing: 29.10.1997
(51) Int. Cl.: A61L 2/10, A61L 2/20, A61L 2/18, B65B 55/04

(54) **Method and apparatus for sterilizing a packaging sheet material**
Verfahren und Vorrichtung zum Sterilisieren von Packstoffbahnen
Procédé et appareil pour stériliser des matériaux d'emballage en feuille

(43) Date of publication of application: 02.06.1999
(73) Proprietor: Tetra Laval Holdings & Finance SA, 1009 Pully (CH)
(72) Inventor: Moruzzi, Guido, 40068 S. Lazzaro Di Savena (IT)
(74) Representative: Cerbaro, Elena, Dr.

(56) References cited:
- EP-A- 0 361 858
- WO-A-97/35768
- US-A- 4 225 556
- US-A- 4 289 728
- US-A- 4 366 125
- US-A- 4 375 145
- PATENT ABSTRACTS OF JAPAN vol. 014, no. 135 (M-0949), 14 March 1990 & JP 02 004621 A (SNOW BRAND MILK PROD CO LTD;OTHERS: 01), 9 January 1990,
- DATABASE WPI Section Ch, Week 8707 Derwent Publications Ltd., London, GB; Class D22, AN 87-046573 XP002061293 & JP 62 004 038 A (DAINIPPON PRINTING CO LTD) , 10 January 1987

## Description

### TECHNICAL FIELD.

The present invention relates to an improved method and apparatus for sterilizing a packaging sheet material of the kind used for packaging pourable food products such as milk, tomato puree, yoghurt, fruit juices, wine, tea, etc. Such laminated packaging sheet material has a multi-layered structure including a fibre-based layer made of a material such as paper, which is coated on either side with a heat-sealable plastics material such as polyethylene. When the packaging material is to be used for the aseptic packaging of pourable products such as milk treated at ultra-high temperature (UHT milk), the face of the packaging material destined to be placed in contact with the food product is also provided with a layer of barrier material, such as e.g., aluminium, which is in turn covered with a layer of plastics material such as polyethylene.

When manufacturing packages from the above-mentioned kind of packaging sheet material, it is imperative that no microorganisms are allowed to come into contact with the food product to be packaged. Therefore, the packaging material must be sterilized immediately prior to use for the manufacture of packages, and it must be retained in sterile conditions, until the package is completely sealed, thereby ensuring that the food product contained therein is free from any microorganisms which would otherwise spoil the food and/or transmit diseases to the consumer.

### BACKGROUND ART.

Laminated packaging material may be produced in the form of web which can be continuously fed into a package forming, filling and sealing machine. Such machines include e.g., the TBA/19 ® and TBA/21® filling machine, manufactured by Tetra Brik Packaging Systems, Via Delfini 1, Modena, Italy. The web is sterilized with a sterilizing agent such as e.g., hydrogen peroxide, which is thereafter removed by evaporation. The sterile packaging material is then maintained in an aseptic chamber, then longitudinally sealed to form a continuous tube, which is filled with liquid food product to be packaged. The tube is then clamped and transversely sealed at regular intervals to produce pillow-shaped packages, which are mechanically folded to produce finished packages. Such types of packages include e.g., parallelepiped packages known by the registered trademarks Tetra Brik Aseptic ® and Tetra Brik ®, and substantially parallelepiped packages with bevelled corners commonly known by the registered trademarks Tetra Prisma ® and Tetra Prisma Aseptic ®.

Alternatively, the laminated packaging sheet material may be cut into blanks and then formed into packages on mandrels. Such packages are sterilized by spraying with hydrogen peroxide. The spray produces a thin layer of hydrogen peroxide on the hydrophobic, thermoplastic outer layer of the packaging sheet material. Successively, a source of UV light in the 200-325nm range is irradiated onto the hydrogen peroxide-covered outer layer of packaging material. The synergy occurring between the hydrogen peroxide and the UV light has a killing effect on any microorganisms on the packaging material. Thereafter, the hydrogen peroxide is removed and the packages are filled with liquid food product and sealed to produce gable-top container commonly known by the registered trademark Tetra Rex ®.

### Sterilization with hydrogen peroxide:

In the sterilization systems employing hydrogen peroxide, without any irradiation thereon of UV light, efforts have been made to prolong the time that the hydrogen peroxide remains in contact with the packaging material, in order to increase the killing effect of the sterilization process. US-A-3,904,361 describes a process wherein, in order to prevent or at least minimize evaporation of a peroxide film on a packaging material web during its passage through a sterilization chamber, to prolong its contact with the web face and obtain the desired sterilization, the chamber is saturated with steam and hydrogen peroxide vapours obtained by spraying hydrogen peroxide through nozzles into the chamber. Although this technique prolonged contact of a very hot film of hydrogen peroxide with the packaging web surface to improve sterilization, it imposed severe restrictions on the rate at which packages could be manufactured with the packaging machine.

Another problem encountered with the known sterilization techniques employing hydrogen peroxide, regards ensuring uniformity of the degree of sterilization throughout the packaging material. In some web-fed packaging machines, hydrogen peroxide was mixed with water and delivered drop-wise into a heated container placed in the longitudinally sealed tube of packaging material. However, the vaporized hydrogen peroxide only started to have a sterilizing effect when the major part of the water in the droplets had evaporated. Therefore, the concentration of hydrogen peroxide actually acting on the packaging material varied considerably, at the same rate as delivery of drops of sterilizing agent. In order to overcome this problem US-A-4,225,556 disclosed the use of a first treatment station with a container for hydrogen peroxide through which the packaging material was passed, and a second treatment station comprising a chamber with an inlet and an outlet for the packaging material web, and a nozzle for spraying hydrogen peroxide onto a heated surface. However, neither this arrangement, nor the above-described sterilization technique known from US-A-3904361 are compatible with modern high-speed liquid food packaging machines due to the excessive times required to effect sterilization.

In order to overcome problems regarding uniformity of the sterilization of a packaging material web with hydrogen peroxide, the packaging material is passed through a bath containing hydrogen peroxide sterilizing solution, and in order to cope with ever-increasing production rates, the hydrogen peroxide baths have been increased in size to maintain contact time between the packaging material and the sterilizing solution. The intensified contact between the hydrogen peroxide solution and the packaging material is of course an advantage from the point of view of bacteriological killing. However, the intensified contact also increases the risk that the hydrogen peroxide solution will penetrate into and degrade the liquid-absorbent fibrous layer of the packaging material web through the cut edges of the web. The over-dimensioning of the bath also results in a further increase in hydrostatic pressure which further increases the risk of liquid penetrating into the web in the deeper parts of the bath. Furthermore, since packaging material located in the bath during machine stoppages is discarded, the deeper baths caused greater wastage of material upon each machine stop.

### Sterilization with hydrogen peroxide and UV radiation:

US-A-4,289,728 to Peel et al dealt with the synergy that exists between UV radiation below 325nm, and hydrogen peroxide at a concentration of at least 0.01% and no more than 10% by weight. By treating a microorganism at the surface of the packaging with an ultraviolet irradiated solution of hydrogen peroxide, the microorganism is rendered non-viable by the synergism between the radiation and the hydrogen peroxide.
However, even with the use of powerful UV lamps, a long irradiation time was required for thorough sterilization, and this long irradiation time gave rise to damaging of the packaging material, lowering of its heat-seal strength and discoloration thereof. As a solution to this problem, US-A-4,366,125 to Kodera et al disclosed a system including a first station for applying a thin film of hydrogen peroxide at low concentration at room temperature onto the outer surfaces of a packaging material. A second station, located downstream of the first station with respect to a travel direction defined by the material to be sterilized, was provided for irradiating the outer hydrogen peroxide-coated surfaces with UV radiation. Finally, a third station was provided downstream of the second station for drying the material with aseptic hot air. US-A-5,114,670 to Duffey disclosed a sterilization chamber including inlet and outlet means for the material to be sterilized, means for introducing gaseous hydrogen peroxide into the sterilization chamber, and means for simultaneously irradiating the material to be sterilized with UV energy. However all of the above-mentioned sterilization techniques are susceptible to improvement relating to the killing effect achieved and the time necessary to guarantee elimination of all pathogenic microorganisms, on the packaging material, for compatibility with modern high-speed liquid food packaging machines which can form, fill and seal 18000 or even more aseptic packages per hour, and wherein the packaging material moves at a speed of, or greater than 81.65 cm per second (48.990 meters per minute), such as the package forming, filling and sealing machine described in EP-A-97830312.1, filed on June 27, 1997, by the same applicant.

### OBJECTS OF THE INVENTION.

There is a general need in the art to provide a method and apparatus for sterilizing packaging sheet material, which overcomes the problems encountered in the prior art sterilization methods and apparatuses.
A main object of the invention is to provide a method and apparatus for sterilizing packaging sheet material, which achieves improved killing rates with respect to the known sterilization techniques, thereby improving the quality of sealed packages manufactured with the packaging sheet material and hence the product delivered to the consumer.

Another object of the invention is to provide a method and apparatus for sterilizing packaging sheet material which is fully compatible with modern high-speed package forming, filling and sealing machines.

A further object of the invention is to provide a method and apparatus for sterilizing packaging sheet material employing hydrogen peroxide, which minimizes the time that the sheet material remains in contact with the hydrogen peroxide, thereby avoiding any soaking of the hydrogen peroxide into the cut edge of the packaging sheet material and permitting, when a hydrogen peroxide bath is used, to construct a bath of reduced depth, whereby no problems arise relating to hydrostatic pressures which would otherwise tend to promote soaking of the hydrogen peroxide into the cut edge of the packaging sheet material.

Yet another object of the invention is to provide a method and apparatus for sterilizing packaging sheet material employing hydrogen peroxide and UV radiation, wherein commercially available means for generating UV radiation can be used, at a power level which does not have any detrimental effects on the packaging material.

A further object of the invention is to provide a method and apparatus for sterilizing packaging sheet material employing hydrogen peroxide and UV radiation, which can be integrated with current package forming, filling and sealing machines.

### DISCLOSURE OF THE INVENTION.

With the above-mentioned objects in view, as well as other objects of the invention which will become apparent hereinafter, the invention provides a method of sterilizing a packaging sheet material, comprising the steps of;
- applying hydrogen peroxide to a packaging sheet material, and;
- irradiating the packaging sheet material with light including at least one UV wavelength between about 200nm and 320nm,
**characterized in that** it comprises the intermediate step of removing excess hydrogen peroxide from the packaging sheet material, after the step of applying hydrogen peroxide and before the step of irradiating the packaging material, whereby residual hydrogen peroxide absorbed by or located adjacent to any microorganisms present on said packaging sheet material is directly targeted with UV radiation.

According to another aspect of the present invention, there is also provided an apparatus for sterilizing a packaging sheet material which comprises
- means for applying hydrogen peroxide to a packaging sheet material moving in an advancement direction,
- means for irradiating the packaging sheet material with light including at least one UV wavelength between 200nm and 320nm, arranged downstream of said means for applying hydrogen peroxide, with respect to said advancement direction, and;
- means for removing hydrogen peroxide from the packaging sheet material,
**characterized in that** said means for removing hydrogen peroxide from the packaging sheet material are interposed between said means for applying hydrogen peroxide and said means for irradiating the packaging material with light including at least one UV wavelength between 200nm and 320nm, whereby residual hydrogen peroxide absorbed by or located adjacent to any microorganisms present on said packaging sheet material is directly targeted with UV radiation.

### BRIEF DESCRIPTION OF THE DRAWINGS.

Further features and advantages of the invention will become apparent from the following detailed description of the invention, and the accompanying drawing figures wherein:
Figure 1 is a schematic view of the apparatus according to the invention;
Figure 2 is an enlarged, schematic cross-sectional view of a portion of packaging sheet material irradiated with UV light according to the methods of the prior art, and;
Figure 3 is an enlarged, schematic cross-sectional view of a portion of packaging sheet material irradiated with UV light according to the method of the present invention.

### DESCRIPTION OF THE PREFERRED EMBODIMENTS.

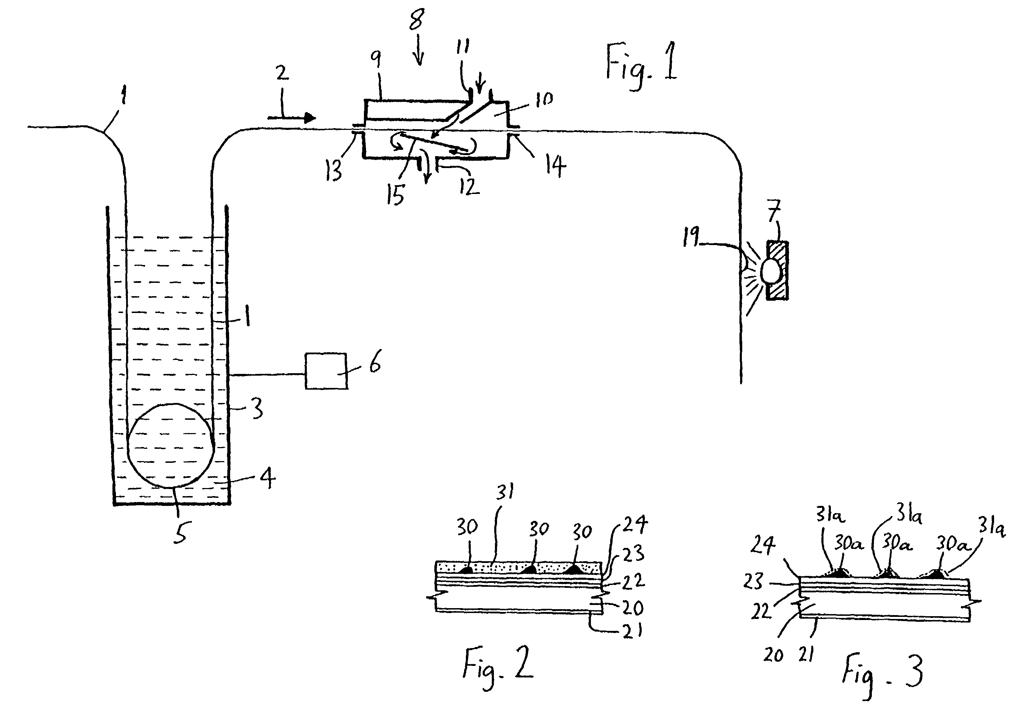

With reference to drawing figure 1, the apparatus according to the invention is shown together with a packaging material sheet to be sterilized. Although, in the illustrated example, the packaging material is in the form of a web 1, it will be appreciated that the material may also be in the form of a cut blank. As shown in figure 3, the web 1 is a laminated multi-layer material of the type commonly used for aseptic packaging of pourable food products. The laminated structure has a fibre-based layer 20 made of a material such as paper, and heat-sealable layers 21, 22 made of plastics material such as polyethylene, provided on each side of the fibre-based layer 20. When the packaging material is to be used for the aseptic packaging of pourable products such as milk treated at ultra-high temperature (UHT milk), the face of the packaging material destined to be placed in contact with the food product is also provided with a layer of barrier material 23, such as e.g., aluminium, which is in turn covered with an additional heat-sealable layer 24 of plastics material such as polyethylene.
The web 1 is moved in an advancement direction, indicated by the arrow 2, and guided in its trajectory, by conventional means which do not form part of the present invention and thus are not illustrated. As clearly shown in figure 1, the apparatus according to the invention includes means for applying hydrogen peroxide to the packaging sheet material 1, moving in the advancement direction 2, which are preferably constituted by a bath 3 containing liquid hydrogen peroxide 4. The hydrogen peroxide 4 in the bath 3 may have a concentration of up to 50% by weight, and preferably has a concentration of from 10% by weight to 50% by weight, and most preferably has a concentration of from 20% by weight to 40% by weight. A conventional roller 5 is provided in the bath for guiding the packaging material 1. As also schematically shown in figure 1, means 6 are also connected to the bath 3 for maintaining the hydrogen peroxide at a temperature preferably comprised between 15 degrees Centigrade and 80 degrees Centigrade, and may be constituted by a conventional thermostatically-controlled heating mechanism 6 as commonly used in the technical field of the invention, such as those present on the above cited TBA/19 ® and TBA/21 ® liquid food packaging machines, available from Tetra Brik Packaging Systems, Via Delfini, Modena, Italy.
In the apparatus according to the invention, the depth of the hydrogen peroxide bath can be reduced with respect to conventional baths, and the hydrogen peroxide 4 in the bath 3 preferably defines a liquid column having a height less than 50cm. One skilled in the art will appreciate that other means for applying hydrogen peroxide liquid or vapour to the surface of the packaging material may also be used, such as spray means.
Means for irradiating the packaging sheet material with light, including at least one UV wavelength between 200nm and 320nm, are located downstream of the bath 4, with respect to the advancement direction 2, and comprise a UV light source 7. According to a preferred embodiment of the invention, the UV light source 7 for irradiating the packaging sheet material may comprises a monochromatic source of UV light having a wavelength of 222nm. The monochromatic source of UV light having a wavelength of 222nm preferably comprises at least one excimer lamp. Such lamps are available from Heraeus Noblelight, Kleinostheim, Germany. Alternatively, a source of polychromatic UV light may also be used. Such polychromatic lamps are manufactured, for instance, by Berson UV-techniek, De Huufkes 23, NL-5674Nuenen, The Netherlands. Other lamps or lasers may also be used which emit light in the UV wavelengths.

As shown in figure 1, means 8 are also provided for removing hydrogen peroxide from the packaging sheet material 1. In the illustrated example, the means 8 for removing hydrogen peroxide from the packaging sheet material preferably comprise at least one air knife 9 for blowing air onto the packaging material sheet 1. Such air knives are known from US-A-4603490 to Hilmersson et al, and have a chamber 10 including an air inlet 11 and an air outlet 12 for a flow of air, a material inlet 13 and a material outlet 14 for a packaging material web 1, and a baffle plate 15 disposed over the air outlet 12 at an angle in relation to the path of travel of the web 1 through the chamber 10. Air blown onto the web 1 at the air knife is preferably heated to a temperature of from 80 degrees Centigrade to 150 degrees Centigrade. It will be appreciated that other means for removing the hydrogen peroxide from the web 1 may also be used. For example, conventional squee-gee rollers or pinch rollers, may also be used in addition to or instead of the air knife. Other means may also be used for generating a flow of hot air suitable for evaporating the hydrogen peroxide. However, means for blowing hot air onto the packaging material web is preferred, especially where the web has any parts, such as package opening devices injection-molded directly onto the web, which could affect the efficiency of the squee-gee rollers.
An important feature of the present invention resides in the specific location of the means for removing excess hydrogen peroxide from the surface of the packaging material web 1. As clearly shown in figure 1, the means for removing excess hydrogen peroxide 8 are interposed between the means for applying hydrogen peroxide, constituted in the illustrated example by the bath 3, and the means 7 for irradiating the packaging material with light including at least one UV wavelength between 200nm and 320nm. The reason for this location of the means for removing hydrogen peroxide is the following:
With reference first of all to drawing figure 2, there is illustrated an enlarged sectional view of a portion of a multi-layer laminated packaging sheet material like the above-described web 1, wherein identical layers of the laminated structure are identified by the same reference numerals. Additionally, the reference numeral 30 schematically indicates microorganisms present on the surface of the packaging material, and the reference numeral 31 indicates the layer of hydrogen peroxide applied to the surface of the packaging material.
Figure 2 illustrates the treatment of a packaging sheet material according to the prior art methods, wherein hydrogen peroxide at a concentration no greater than 10% by weight is first applied to the packaging sheet material. Thereafter the hydrogen peroxide is irradiated with UV light, and the synergy between the hydrogen peroxide and the UV light has the known killing effect on the microorganisms 30.
Figure 3 illustrates the treatment of a packaging sheet material according to the invention, wherein hydrogen peroxide, preferably at a concentration of up to 50% by weight, and preferably of from 10% to 50% by weight, is first applied to the packaging sheet material. Thereafter the hydrogen peroxide is removed from the surface of the packaging material. It would thus appear that if one irradiated the packaging material with UV light at this point, i.e., in the UV irradiation zone indicated by the reference numeral 19 in figure 1, there could be no interaction between the UV light and hydrogen peroxide, because the latter has been removed. However, the applicant has discovered that on the contrary, the killing effect achieved is significantly improved with respect to known techniques. This is due to the fact that although excess hydrogen peroxide is removed from the hydrophobic packaging sheet material, a residual or trace quantity is retained at any microorganisms 30a, which are believed to hydrophilically absorb the hydrogen peroxide 31a or otherwise retain the residual hydrogen peroxide. Therefore, when the packaging sheet material is successively irradiated with UV light, the interaction occurring between the hydrogen peroxide and the UV light, that has the known killing effect on microorganisms, is targeted specifically to the microorganisms themselves, which have hydrophilically absorbed or otherwise retained the hydrogen peroxide. Furthermore, the removal of the shielding layer of excess hydrogen peroxide allows advantage to be taken of the possibility of using higher concentrations of hydrogen peroxide than those which were considered to be the maximum utilizable by the prior art.
In other words, instead of irradiating the entire layer of hydrogen peroxide with the aim of killing any microorganisms contained therein, as taught by the prior art, in accordance with the invention, the layer of excess hydrogen peroxide 31 is removed and only the residual hydrogen peroxide located in, on or adjacent to any microorganisms 30a present on the surface of the packaging material is directly targeted with UV radiation. It will be understood that the positional relationship between the residual hydrogen peroxide and the microorganisms shown in figure 3 is purely schematic and representative of what is believed to be occurring.
Therefore, when irradiating the packaging material with UV light, instead of having a layer of excess hydrogen peroxide shielding the microorganisms, the irradiation of UV light is targeted to the residual hydrogen peroxide absorbed by or located adjacent to the microorganisms. The removal of the layer of excess hydrogen peroxide in practice has the surprising effect of significantly increasing the efficiency of the sterilization process.

The method according to the invention, carried out with the above-described apparatus, will now be described:
First of all, hydrogen peroxide, preferably liquid hydrogen peroxide at a concentration of up to 50% by weight, preferably of from 10% by weight to 50% by weight, and most preferably at a concentration of from 20% by weight to 40% by weight, is applied to a to a packaging sheet material. In accordance with a preferred embodiment of the invention, this is achieved by immersing the packaging sheet material in a hydrogen peroxide bath at a temperature comprised between 15 degrees Centigrade and 80 degrees Centigrade, for a time interval of from 0.5 seconds to 2 seconds. During this time, the hydrogen peroxide is believed to be hydrophilically absorbed in, or become somehow entrapped adjacent to or on any microorganisms present on the packaging material. Preferably, the height of the liquid column of hydrogen peroxide in the bath does not exceed 50cm above the packaging sheet material. Although deeper hydrogen peroxide baths may be used, this relatively short residence time and shallow depth obviate problems relating to edge-soaking or wicking of the hydrogen peroxide into the fiberous layer of the laminated packaging material.
Thereafter, the excess hydrogen peroxide is removed from the surface of the packaging sheet material, preferably by blowing thereon a stream of air heated to a temperature of from 80 degrees Centigrade to 150 degrees Centigrade. This removes the excess hydrogen peroxide from the packaging material, but a trace quantity of residual hydrogen peroxide is retained at any microorganisms present on the surface of the packaging material.
Once the excess hydrogen peroxide has been removed from the surface of the packaging material, the material is irradiated with light including at least one UV wavelength between about 200nm and 320nm. According to a preferred embodiment of the invention, the packaging sheet material is irradiated with UV light at a wavelength of 222nm, and most preferably, the UV light source is an excimer lamp. In this manner, it has been found that it is possible to directly target residual hydrogen peroxide entrapped at any microorganisms present on the packaging sheet material with UV radiation. The use of an excimer lamp has the additional advantages of instantaneous activation and deactivation and no significant heat emission.

The sterilization system according to the invention is fully compatible with modern high-speed aseptic liquid-food packaging machines, producing up to 18000 or more packages per hour, wherein the packaging material moves at a speed of, or greater than 81.65 cm per second (48.990 meters per minute).

The present invention may be further modified, without thereby departing from the purview of the appended claims.

## Claims

1. Method of sterilizing a packaging sheet material, comprising the steps of;
- applying hydrogen peroxide to a packaging sheet material, and;
- irradiating the packaging sheet material with light including at least one UV wavelength between about 200nm and 320nm,
**characterized in that** it comprises the intermediate step of removing excess hydrogen peroxide from the packaging sheet material, after the step of applying hydrogen peroxide and before the step of irradiating the packaging material, whereby residual hydrogen peroxide absorbed by or located adjacent to any microorganisms present on said packaging sheet material is directly targeted with UV radiation.

2. Method according to claim 1, **characterized in that** said step of applying hydrogen peroxide to said packaging sheet material comprises applying liquid hydrogen peroxide thereto at a concentration of up to 50% by weight.

3. Method according to claim 1, **characterized in that** said step of applying hydrogen peroxide to said packaging sheet material, comprises applying liquid hydrogen peroxide at a concentration of from 20% by weight to 40% by weight.

4. Method according to claim 1, 2 or 3, **characterized in that** said step of applying hydrogen peroxide to said packaging sheet material comprises the step of immersing said packaging sheet material in a hydrogen peroxide bath at a temperature comprised between 15 degrees Centigrade and 80 degrees Centigrade, for a time interval of from 0.5 seconds to 2 seconds.

5. Method according to claim 1, **characterized in that** said intermediate step of removing excess hydrogen peroxide from said packaging sheet material comprises blowing a stream of heated air, heated to a temperature of from 80 degrees Centigrade to 150 degrees Centigrade onto said packaging sheet material.

6. Method according to claim 1, **characterized in that** said step of irradiating the packaging sheet material with light including at least one UV wavelength, consists of irradiating said packaging sheet material with polychromatic UV light.

7. Method according to claim 1, **characterized in that** said step of irradiating the packaging sheet material with light including at least one UV wavelength, consists of irradiating said packaging sheet material with UV light at a wavelength of 222nm.

8. Method according to claim 7, **characterized in that** said step of irradiating the packaging sheet material with UV light at a wavelength of 222nm comprises irradiating said packaging sheet material with an excimer lamp.

9. Method according to one or more of claims 1-8, **characterized in that** said packaging sheet material is a web unwound from a roll.

10. Method according to one or more of claims 1-8, **characterized in that** said packaging sheet material is a blank.

11. Apparatus for sterilizing a packaging sheet material according to the method defined in claims 1-10, comprising;
- means (3) for applying hydrogen peroxide to a packaging sheet material (1) moving in an advancement direction,
- means (7) for irradiating the packaging sheet material with light including at least one UV wavelength between 200nm and 320nm, arranged downstream of said means for applying hydrogen peroxide, with respect to said advancement direction, and;
- means (8) for removing excess hydrogen peroxide from the packaging sheet material,
**characterized in that** said mean (8) for removing hydrogen peroxide from the packaging sheet material are interposed between said means (3) for applying hydrogen peroxide and said means (7) for irradiating the packaging material with light including at least one UV wavelength between 200nm and 320nm, whereby residual hydrogen peroxide absorbed by or located adjacent to any microorganisms present on said packaging sheet material is directly targeted with UV radiation.

12. Apparatus according to claim 11, **characterized in that** said means for applying hydrogen peroxide to said packaging sheet material comprise a bath containing liquid hydrogen peroxide at a concentration of up to 50% by weight.

13. Apparatus according to claim 11, **characterized in that** said means for applying hydrogen peroxide to said packaging sheet material comprise a bath containing liquid hydrogen peroxide at a concentration of from 20% by weight to 40% by weight.

14. Apparatus according to claim 12 or 13, **characterized in that** it comprises means for maintaining said hydrogen peroxide bath at a temperature comprised between 15 degrees Centigrade and 80 degrees Centigrade.

15. Apparatus according to claim 12, 13 or 14, **characterized in that** said hydrogen peroxide in said bath defines a liquid column having a height less than 50cm in said bath.

16. Apparatus according to claim 11, **characterized in that** said means for removing hydrogen peroxide from said packaging sheet material comprise at least one air knife for blowing air onto said packaging material sheet at a temperature of from 80 degrees Centigrade to 150 degrees Centigrade.

17. Apparatus according to claim 11, **characterized in that** said means for irradiating the packaging sheet material with light include at least one UV wavelength between about 200nm and 320nm comprise a monochromatic source of UV light having a wavelength of 222nm.

18. Apparatus according to claim 17, **characterized in that** said monochromatic source of UV light comprises at least one excimer lamp.

19. Apparatus according to claim 11, **characterized in that** said means for irradiating the packaging sheet material with light including at least one UV wavelength, comprise a polychromatic UV lamp.

## Patentansprüche

1. Verfahren zum Sterilisieren einer Packstoffbahn mit den Verfahrensstufen
Aufbringen von Wasserstoffperoxid auf die Packstoffbahn und Bestrahlen der Packstoffbahn mit Licht einschl. mindestens einer UV-Wellenlänge zwischen etwa 200 nm und 320 nm,
**dadurch gekennzeichnet,**
**daß** das Verfahren den Zwischenschritt des Entfernens bzw. Abführens überschüssigen Wasserstoffperoxids von der Packstoffbahn nach der Verfahrensstufe des Aufbringens von Wasserstoffperoxid und vor der Verfahrensstufe des Bestrahlens der Verpackungsbahn einschließt, wodurch Restwasserstoffperoxid, der durch irgendwelche an der Packstoffbahn befindlichen Mikroorganismen absorbiert oder in der Nähe derselben verblieben ist, unmittelbar mit UV-Bestrahlung getroffen wird.

2. Verfahren nach Anspruch 1,
**dadurch gekennzeichnet,**
**daß** die Verfahrensstufe des Aufbringens von Wasserstoffperoxid auf die Packstoffbahn das Aufbringen von flüssigem Wasserstoffperoxid auf dieser mit einer Konzentration von bis zu 50 Gew.-% einschließt.

3. Verfahren nach Anspruch 1,
**dadurch gekennzeichnet,**
**daß** die Verfahrensstufe des Aufbringens von Wasserstoffperoxid auf die Packstoffbahn das Aufbringen von flüssigem Wasserstoffperoxid mit einer Konzentration zwischen 20 und 40 Gew.-% einschließt.

4. Verfahren nach einem der Ansprüche 1, 2 und 3,
**dadurch gekennzeichnet,**
**daß** die Verfahrensstufe des Aufbringens von Wasserstoffperoxid auf die Packstoffbahn eine Verfahrensstufe des Eintauchens der Packstoffbahn in ein Wasserstoffperoxidbad bei einer Temperatur zwischen 15°C und 80°C für eine Zeitdauer zwischen 0,5 Sekunden und 2 Sekunden einschließt.

5. Verfahren nach Anspruch 1,
**dadurch gekennzeichnet,**
**daß** der Zwischenschritt des Entfernens überschüssigen Wasserstoffperoxids bzw. von Restwasserstoffperoxid von der Packstoffbahn das Aufblasen eines Stromes erhitzter Luft auf die Packstoffbahn einschließt, welche auf eine Temperatur zwischen 80°C und 150°C erhitzt ist.

6. Verfahren nach Anspruch 1,
**dadurch gekennzeichnet,**
**daß** die Verfahrensstufen des Bestrahlens der Packstoffbahn mit Licht einschl. mindestens einer UV-Wellenlänge die Bestrahlung der Packstoffbahn mit polychromatischem UV-Licht enthält.

7. Verfahren nach Anspruch 1,
**dadurch gekennzeichnet,**
**daß** die Verfahrensstufe des Bestrahlens der Packstoffbahn mit Licht einschl. mindestens einer UV-Wellenlänge das Bestrahlen der Packstoffbahn mit UV-Licht einer Wellenlänge von 222 nm enthält.

8. Verfahren nach Anspruch 7,
**dadurch gekennzeichnet,**
**daß** die Verfahrensstufe des Bestrahlens der Packstoffbahn mit UV-Licht einer Wellenlänge von 222 nm das Bestrahlen der Packstoffbahn mit einer Excimerlampe einschließt.

9. Verfahren nach einem oder mehreren der Ansprüche 1-8,
**dadurch gekennzeichnet,**
**daß** für die Packstoffbahn eine von einer Rolle abgewickelte Bahn verwendet wird.

10. Verfahren nach einem oder mehreren der Ansprüche 1-8,
**dadurch gekennzeichnet,**
**daß** für die Packstoffbahn ein Zuschnitt verwendet wird.

11. Vorrichtung zum Sterilisieren einer Packstoffbahn nach dem Verfahren nach einem der Ansprüche 1 bis 10 mit folgenden Bestandteilen:
Eine Aufbringungseinrichtung (3) zum Aufbringen von Wasserstoffperoxid auf die in einer Vorschubrichtung bewegte Packstoffbahn (1);
eine Bestrahlungseinrichtung (7) zum Bestrahlen der Packstoffbahn mit Licht einschl. mindestens einer UV-Wellenlänge zwischen 200 nm und 320 nm in Fortschreitungsrichtung hinter der Aufbringeinrichtung für Wasserstoffperoxid und eine Abnahmeeinrichtung (8) zum Entfernen überschüssigen Wasserstoffperoxid von der Packstoffbahn,
**dadurch gekennzeichnet,**
**daß** die Abnahmeeinrichtung (8) zum Entfernen von Wasserstoffperoxid von der Packstoffbahn zwischen der Aufbringungseinrichtung (3) zum Aufbringen von Wasserstoffperoxid und der Bestrahlungseinrichtung (7) zum Bestrahlen der Verpackungsmaterial mit Licht einschl. mindestens einer UV-Wellenlänge zwischen 200 nm und 320 nm angeordnet ist, wodurch überschüssiger bzw. Restwasserstoffperoxid der von auf der Packstoffbahn befindlichen Mikroorganismen absorbiert oder unmittelbar daneben verblieben ist, unmittelbar mit UV-Strahlen bestrahlbar ist.

12. Vorrichtung nach Anspruch 11,
**dadurch gekennzeichnet,**
**daß** die Aufbringungseinrichtung zum Aufbringen von Wasserstoffperoxid auf die Packstoffbahn ein flüssiges Wasserstoffperoxid mit einer Konzentration von bis zu 50 Gew.-% enthaltendes Bad aufweist.

13. Vorrichtung nach Anspruch 11,
**dadurch gekennzeichnet,**
**daß** die Aufbringungseinrichtung zum Aufbringen von Wasserstoffperoxid auf die Packstoffbahn ein flüssigen Wasserstoffperoxid mit einer Konzentration zwischen und 20 und 40 Gew.-% enthaltendes Bad aufweist.

14. Vorrichtung nach Anspruch 12 oder 13,
**dadurch gekennzeichnet,**
**daß** sie eine Einrichtung zum Beibehalten einer Temperatur zwischen 15°C und 80°C des Wasserstoffperoxidbades aufweist.

15. Vorrichtung nach Anspruch 12, 13 oder 14,
**dadurch gekennzeichnet,**
**daß** das Wasserstoffperoxid in dem Bad eine Flüssigkeitssäule mit einer Höhe von weniger als 50 cm im Bad bildet.

16. Vorrichtung nach Anspruch 11,
**dadurch gekennzeichnet,**
**daß** die Abnahmeeinrichtung zum Entfernen von Wasserstoffperoxid von der Packstoffbahn mindestens ein Luftmesser bzw. eine Luftdüse zum Aufblasen von Luft auf die Packstoffbahn bei einer Temperatur zwischen 80°C und 150°C aufweist.

17. Vorrichtung nach Anspruch 11,
**dadurch gekennzeichnet,**
**daß** die Bestrahlungseinrichtung zum Bestrahlen der Packstoffbahn mit Licht einschl. einer UV-Wellenlänge zwischen etwa 200 nm und 320 nm eine monochromatische UV-Lichtquelle mit einer Wellenlänge von 222 nm aufweist.

18. Vorrichtung nach Anspruch 17,
**dadurch gekennzeichnet,**
**daß** die monochromatische UV-Lichtquelle mindestens eine Excimerlampe (insb. gepulster Gaslaser mit intensiver Lichtemission im UV-Spektralbereich) aufweist.

19. Vorrichtung nach Anspruch 11,
**dadurch gekennzeichnet,**
**daß** die Bestrahlungseinrichtung zum Bestrahlen der Packstoffbahn mit Licht einschl. mindestens einer UV-Wellenlänge eine polychromatische UV-Lampe aufweist.

## Revendications

1. Procédé de stérilisation d'un matériau d'emballage en feuille, comprenant les étapes de :
application d'eau oxygénée à un matériau d'emballage en feuille, et
irradiation du matériau d'emballage en feuille avec une lumière Incluant au moins une longueur d'onde dans l'ultraviolet comprise entre 200 nm et 320 nm environ.
**caractérisé en ce qu'**il comprend l'étape intermédiaire d'élimination de l'eau oxygénée en excès du matériau d'emballage en feuille, après l'étape d'application d'eau oxygénée et avant l'étape d'irradiation du matériau d'emballage, afin que l'eau oxygénée résiduelle absorbée par les microorganismes présents sur le dit matériau d'emballage en feuille, ou qui se trouve près de ces derniers, soit directement visée par le rayonnement ultraviolet.

2. Procédé selon la revendication 1, **caractérisé en ce que** la dite étape d'application d'eau oxygénée au dit matériau d'emballage en feuille comprend l'application d'eau oxygénée liquide au dit matériau à une concentration pouvant atteindre 50% en poids.

3. Procédé selon la revendication 1, **caractérisé en ce que** la dite étape d'application d'eau oxygénée au dit matériau d'emballage en feuille comprend l'application d'eau oxygénée liquide à une concentration comprise entre 20% en poids et 40% en poids.

4. Procédé selon la revendication 1, 2 ou 3, **caractérisé en ce que** la dite étape d'application d'eau oxygénée au dit matériau d'emballage en feuille comprend l'étape d'immersion du dit matériau d'emballage en feuille dans un bain d'eau oxygénée à une température comprise entre 15°C et 80°C, pendant un laps de temps de 0,5 seconde à 2 secondes.

5. Procédé selon la revendication 1, **caractérisé en ce que** la dite étape intermédiaire d'élimination de l'eau oxygénée en excès du dit matériau d'emballage en feuille comprend le soufflage d'un courant d'air chaud chauffé à une température de 80°C à 150°C, sur le dit matériau d'emballage en feuille.

6. Procédé selon la revendication 1, **caractérisé en ce que** la dite étape d'irradiation du matériau d'emballage en feuille avec une lumière incluant au moins une lumière d'onde dans l'ultraviolet consiste à irradier le dit matériau d'emballage en feuille avec une lumière ultraviolette polychromatique.

7. Procédé selon la revendication 1, **caractérisé en ce que** la dite étape d'irradiation du matériau d'emballage en feuille avec une lumière incluant au moins une longueur d'onde dans l'ultraviolet consiste à irradier le dit matériau d'emballage en feuille avec une lumière ultraviolette à une longueur d'onde de 222 nm.

8. Procédé selon la revendication 7, **caractérisé en ce que** la dite étape d'irradiation du matériau d'emballage en feuille avec une lumière ultraviolette d'une longueur d'onde de 222 nm comprend l'irradiation du dit matériau d'emballage en feuille avec une lampe excimère.

9. Procédé selon une ou plusieurs des revendications 1 à 8, **caractérisé en ce que** le dit matériau d'emballage en feuille est une bande déroulée d'une bobine.

10. Procédé selon une ou plusieurs des revendications 1 à 8, **caractérisé en ce que** le dit matériau d'emballage en feuille est un flan.

11. Appareil pour la stérilisation d'un matériau d'emballage en feuille selon le procédé défini dans les revendications 1 à 10, comprenant :
des moyens (3) d'application d'eau oxygénée à un matériau d'emballage en feuille (1) qui se déplace dans une direction d'avance,
des moyens (7) d'irradiation du matériau d'emballage en feuille avec une lumière incluant au moins une longueur d'onde dans l'ultraviolet entre 200 nm et 320 nm, placés en aval des dits moyens d'application d'eau oxygénée avec référence à la dite direction d'avance, et
des moyens (8) d'élimination de l'eau oxygénée en excès du matériau d'emballage en feuille,
**caractérisé en ce que** les dits moyens (8) d'élimination de l'eau oxygénée du matériau d'emballage en feuille sont interposés entre les dits moyens (13) d'application d'eau oxygénée et les dits moyens (7) d'irradiation du matériau d'emballage avec une lumière incluant au moins une longueur d'onde dans l'ultraviolet entre 200 nm et 320 nm, de sorte que l'eau oxygénée résiduelle absorbée par les microorganismes présents sur le dit matériau d'emballage en feuille, ou qui se trouve près de ceux-ci, est directement visée par le rayonnement ultraviolet.

12. Appareil selon la revendication 11, **caractérisé en ce que** les dits moyens d'application d'eau oxygénée au dit matériau d'emballage en feuille comprennent un bain contenant de l'eau oxygénée liquide à une concentration jusqu'à 50% en poids.

13. Appareil selon la revendication 11, **caractérisé en ce que** les dits moyens d'application d'eau oxygénée au dit matériau d'emballage en feuille comprennent un bain contenant de l'eau oxygénée liquide à une concentration de 20% en poids à 40% en poids.

14. Appareil selon la revendication 12 ou 13, **caractérisé en ce qu'**il comprend des moyens de maintien du dit bain d'eau oxygénée à une température comprise entre 15°C et 80°C.

15. Appareil selon la revendication 12, 13 ou 14, **caractérisé en ce que** la dite eau oxygénée dans le dit bain définit une colonne de liquide ayant une hauteur inférieure à 50 cm dans le dit bain.

16. Appareil selon la revendication 11, **caractérisé en ce que** les dits moyens d'élimination d'eau oxygénée du dit matériau d'emballage en feuille comprennent au moins une lame d'air pour souffler de l'air sur la dite feuille de matériau d'emballage à une température de 80°C à 150°C.

17. Appareil selon la revendication 11, **caractérisé en ce que** les dits moyens d'irradiation du matériau d'emballage en feuille avec une lumière incluant au moins une longueur d'onde dans l'ultraviolet entre 200 nm et 320 nm environ comprennent une source monochromatique de lumière ultraviolette ayant une longueur d'onde de 222 nm.

18. Appareil selon la revendication 17, **caractérisé en ce que** la dite source monochromatique de lumière ultraviolette comprend au moins une lampe excimère.

19. Appareil selon la revendication 11, **caractérisé en ce que** les dits moyens d'irradiation du matériau d'emballage en feuille avec une lumière incluant au moins une longueur d'onde dans l'ultraviolet comprennent une lampe à lumière ultosviolette polychromatique.
